# EUROPÄISCHE PATENTSCHRIFT

(11) **EP 3 733 217 B1**
(45) Veröffentlichungstag und Bekanntmachung des Hinweises auf die Patenterteilung: **20.07.2022**
(21) Anmeldenummer: 20167030.4
(22) Anmeldetag: 31.03.2020
(51) Int. Cl.: A61L 2/08, B67B 3/00

(54) **VORRICHTUNG UND VERFAHREN ZUM STERILISIEREN VON BEHÄLTNISVERSCHLÜSSEN**
METHOD AND DEVICE FOR STERILISING CONTAINER CLOSURES
DISPOSITIF ET PROCÉDÉ DE STÉRILISATION DE FERMETURES DE RÉCIPIENT

(30) Priorität: 02.04.2019 DE 102019108565
(43) Veröffentlichungstag der Anmeldung: 04.11.2020
(60) Teilanmeldung: 21183091.4
(73) Patentinhaber: Krones AG, 93073 Neutraubling (DE)
(72) Erfinder: Micko, Christoph, 93073 Neutraubling (DE); Söllner, Jürgen, 93073 Neutraubling (DE); Söllner-Wein, Gertrud, 93073 Neutraubling (DE)
(74) Vertreter: Bittner, Bernhard

(56) Entgegenhaltungen:
- EP-A1- 2 161 202
- EP-A1- 2 610 209
- EP-A1- 2 650 022
- WO-A1-2015/093096
- WO-A1-2015/124358
- JP-A- 2011 213 417

## Beschreibung

Die vorliegende Erfindung bezieht sich auf eine Vorrichtung und ein Verfahren zum Sterilisieren von Behältnisverschlüssen. Derartige Vorrichtungen und Verfahren sind aus dem Stand der Technik seit Langem bekannt. Üblicherweise werden zum Sterilisieren von Behältnisverschlüssen flüssige oder gasförmige Sterilisationsmedium wie Peressigsäure oder H₂O₂ eingesetzt. Mit diesen werden die Behältnisverschlüsse beaufschlagt.

In jüngerer Zeit ist man jedoch bestrebt, auf den Einsatz von Chemikalien zu verzichten oder diesen zumindest zu reduzieren. Generell sind daher aus dem Stand der Technik auch Verfahren bekannt, bei denen Behältnisse oder Kunststoffvorformlinge mittels Elektronenstrahlen sterilisiert werden.

Aus der WO 2015/093096 A1 ist dabei beispielsweise eine Bestrahlung von Außenflächen von Flaschenverschlüssen mit einem Elektronenstrahl während des Transports der Flaschenverschlüsse bekannt.

Der vorliegenden Erfindung liegt daher die Aufgabe zugrunde, eine Vorrichtung und ein Verfahren zur Verfügung zu stellen, welche eine Sterilisation von Behältnisverschlüssen erlauben und dabei gleichwohl den Einsatz von chemischen Sterilisationsmitteln reduzieren oder sogar verhindern.

Diese Aufgabe wird erfindungsgemäß durch die Gegenstände der unabhängigen Patentansprüche erreicht. Vorteilhafte Ausführungsformen und Weiterbildungen sind Gegenstand der Unteransprüche.

Eine erfindungsgemäße Vorrichtung zum Sterilisieren von Behältnisverschlüssen weist eine Transporteinrichtung auf, welche die Behältnisverschlüsse entlang eines vorgegebenen Transportpfads transportiert sowie eine Sterilisationseinrichtung, welche dazu geeignet und bestimmt ist, die Behältnisverschlüsse wenigstens abschnittsweise zu sterilisieren.

Erfindungsgemäß weist die Sterilisationseinrichtung eine Ladungsträgerquelle auf, welche Ladungsträger erzeugt und eine Strahlungseinrichtung, welche die Ladungsträger auf eine zu sterilisierende Oberfläche der Behältnisverschlüsse einstrahlt. Weiterhin ist wenigstens ein Element der Sterilisationseinrichtung in einen Innenbereich der Behältnisverschlüsse einführbar.

Unter dem Innenbereich der Behältnisverschlüsse wird dabei derjenige Bereich der Kunststoffverschlüsse verstanden, der von der Umfangswandung der Behältnisverschlüsse umgeben ist und insbesondere derjenige Bereich, der von einem Innengewinde der Behältnisverschlüsse umgeben ist. Insbesondere handelt es sich bei dem Innenbereich der Behältnisverschlüsse um denjenigen Bereich, der einen Abschnitt der Mündung des Behältnisses in einem verschlossenen Zustand des Behältnisses aufnimmt.

Durch das Einführen wenigstens eines Elements der Sterilisationseinrichtung in den Innenbereich der Behältnisverschlüsse ist eine vollständige Sterilisation dieses Innenbereichs und insbesondere auch der Umfangsinnenwandung der Behältnisverschlüsse möglich.

Insbesondere handelt es sich bei diesen Ladungsträgern um Elektronen. Diese können beispielsweise mittels einer Glühkatode erzeugt werden. Es werden jedoch auch andere Ladungsträger denkbar, wie beispielsweise Protonen, Alphateilchen und dergleichen.

Es wird daher vorgeschlagen, eine Sterilisation der Behältnisverschlüsse mittels Elektronenstrahlung vorzunehmen.

Bei einer weiteren bevorzugten Ausführungsform weist die Sterilisationseinrichtung wenigstens einen rohrförmigen oder stangenförmigen Körper auf, innerhalb dessen die Ladungsträger bewegt und/oder beschleunigt werden. Besonders bevorzugt ist wenigstens ein Abschnitt dieses rohrförmigen Körpers in den Innenraum der Behältnisverschlüsse einführbar. Insbesondere ist dieser wenigstens ein Abschnitt in einem Bereich der Behältnisverschlüsse einführbar, der von einem Innengewinde der Behältnisverschlusses umgeben ist.

Dabei ist es möglich, dass dieser rohrförmige Körper entlang seiner Längsrichtung einen im Wesentlichen konstanten Querschnitt aufweist. Es wäre jedoch auch möglich, dass ein Endabschnitt dieses rohrförmigen Körpers einen verringerten Querschnitt aufweist und dieser rohrförmige Körper insbesondere in demjenigen Bereich, der in den Behältnisverschluss eingeführt wird einen verringerten Querschnitt aufweist.

Bei einer weiteren bevorzugten Ausführungsform weist die Vorrichtung eine Beschleunigungseinrichtung auf, welche die Ladungsträger beschleunigt und welche insbesondere die Elektronen beschleunigt.

Bei einer weiteren bevorzugten Ausführungsform weist die Sterilisationseinrichtung eine Strahlungseinrichtung auf, welche gegenüber den Behältnisverschlüssen in einer Bewegungsrichtung bewegbar ist, welche zumindest eine Komponente aufweist, welche senkrecht zu dem Transportpfad der Behältnisverschlüsse ist. Besonders bevorzugt ist diese Bewegungsrichtung senkrecht zu dem Transportpfad der Behältnisverschlüsse. Diese Strahlungseinrichtung kann dabei am Ende des oben genannten rohrförmigen Körpers angeordnet sein.

Dabei ist es insbesondere möglich, dass die Sterilisationseinrichtung und insbesondere die Strahlungseinrichtung auf die Behältnisverschlüsse zustellbar ist und besonders bevorzugt in den Innenraum dieser Behältnisverschlüsse einführbar ist. Auf diese Weise ist auch eine zuverlässige Sterilisierung des Innengewindebereichs der Behältnisverschlüsse möglich.

Bevorzugt handelt es sich bei den Behältnisverschlüssen um Kunststoffverschlüsse.

Bei einer bevorzugten Ausführungsform weist die Vorrichtung eine Antriebseinrichtung auf, welche die Bewegung der Strahlungseinrichtung auslöst. Es wäre jedoch auch möglich, dass die Behältnisverschlüsse selbst auf die Sterilisationseinrichtung zu bewegt werden. besonders bevorzugt weist die Vorrichtung wenigstens einen Elektromotor auf, der die besagte Bewegung der Strahlungseinrichtung realisiert. Die rohrförmigen Körper werden im Folgenden auch als Strahlfinger bezeichnet.

Bei einer besonders bevorzugten Ausführungsform weisen die rohrförmigen Körper bzw. Strahlfinger ein Austrittsfenster auf, über welches die Ladungsträger und insbesondere die Elektronen aus den rohrförmigen Körpern austreten können. Bei diesem Austrittsfenster kann es sich beispielsweise um ein Titanfenster handeln. Besonders bevorzugt weist dieses Austrittsfenster eine Dicke auf, die größer ist als 1 µm, bevorzugt größer als 2µm, bevorzugt größer als 3 µm, bevorzugt größer als 5 µm. Besonders bevorzugt weist das Austrittsfenster eine Dicke auf, die kleiner ist als 50 µm, bevorzugt kleiner als 40 µm, bevorzugt kleiner als 30 µm, bevorzugt kleiner als 25 µm, bevorzugt kleiner als 20 µm.

Bei einer weiteren bevorzugten Ausführungsform ist wenigstens ein Element der Sterilisationseinrichtung in den Innenbereich der Behältnisverschlüsse einführbar. Insbesondere handelt es sich hier um einen Endabschnitt des oben erwähnten Strahlfingers und insbesondere den Bereich, der auch das besagte Austrittsfenster enthält.

Durch das Einführen des Strahlfingers in diesen Innenbereich können auch die Bereiche des Innengewindes zuverlässig sterilisiert werden. Insbesondere kommt es nicht zu einer Verschattung der einzelnen Bereiche des Innengewindes.

Bei einer weiteren bevorzugten Ausführungsform weist die Sterilisationseinrichtung eine Vielzahl von entlang des Transportpfads hintereinander angeordneten rohrförmigen Körpern bzw. Strahlfingern auf. Auf diese Weise können mehrere hintereinander angeordnete Behältnisverschlüsse mittels des Strahlfingers sterilisiert werden.

Bei einer weiteren bevorzugten Ausführungsform ist die Sterilisationseinrichtung stationär entlang des Transportpfads der Behältnisverschlüsse angeordnet. Hierunter ist zu verstehen, dass sich die Sterilisationseinrichtung und/ oder die Strahlfinger zwar auf die Behältnisverschlüsse zu und von diesen weg bewegen können, das heißt insbesondere senkrecht zu dem Transportpfad, sich aber ansonsten nicht in der Transportrichtung bewegen.

Auf diese Weise kann eine höhere Zuverlässigkeit insbesondere der Sterilisationseinrichtungen erreicht werden, da es sich hierbei um relativ empfindliche Bauteile handelt. Bei der Anmelderin sind bereits intern mehrere technische Ansätze bekannt, um Behältnisverschlüsse mit Elektronen zu sterilisieren. Diese Vorgehensweise basieren jeweils auf dem Prinzip, dass die Behältnisverschlüsse an einem stationär aufgebauten Strahlungsemitter vorbeitransportiert und dadurch sterilisiert werden. Aufgrund des Transportprinzips wirken allerdings die Gewindegänge der Verschlüsse als Schatten für die Bestrahlung der Elektronen und es konnte teilweise kein ausreichender Sterilisationseffekt erreicht werden.

Im Rahmen der vorliegenden Erfindung wird insbesondere eine Möglichkeit vorgeschlagen, die Behältnisverschlüsse dennoch vollumfänglich zu bestrahlen, wobei man die Strahlungsquelle in den Verschluss eintaucht.

Bei einer Ausgestaltung ist es möglich, dass der Transport der Behältnisverschlüsse getaktet erfolgt. So könnte eine bestimmte Anzahl an Behältnisverschlüssen beispielsweise unter die Sterilisationseinrichtungen gefahren werden, anschließend der Transport angehalten werden und die Sterilisationsfinger in die Behältnisverschlüsse eintauchen. Nachdem dies geschehen ist, können diese Behältnisverschlüsse aus dem Sterilisationsbereich gefahren werden und eine neue Charge an Behältnisverschlüssen in den Sterilisationsbereich eingefahren werden.

Es wäre jedoch auch möglich, dass die Sterilisationseinrichtung und/ oder die Sterilisationseinrichtungen beweglich in der Transportrichtung der Behältnisverschlüsse angeordnet sind. So könnten beispielsweise die Sterilisationseinrichtungen auf einem drehbaren Träger befestigt sein, wobei an diesem drehbaren Träger auch Halteeinrichtungen zum Halten der Behältnisverschlüsse angeordnet sind, sodass während der Drehung der Behältnisverschlüsse und auch der Sterilisationseinrichtungen die Sterilisation der Behältnisverschlüsse erfolgt.

Bei einer weiteren bevorzugten Ausführungsform weist die Vorrichtung auch eine Sterilisationseinrichtung auf, welche die Außenoberflächen der Behältnisverschlüsse sterilisiert. Bevorzugt arbeitet auch diese weitere Sterilisationseinrichtung mit Ladungsträgerstrahlung und insbesondere mit Elektronenstrahlung. Dabei kann jedoch diese weitere Sterilisationseinrichtung in der Weise ausgeführt sein, dass die Behältnisverschlüsse an dieser vorbeigeführt werden, da im Außenbereich der Behältnisverschlüsse eine geringere Gefahr an Verschattungen besteht und die Außenoberfläche auch hinsichtlich der Sterilisation weniger kritisch ist als die Innenoberfläche der Behältnisverschlüsse.

Bei einer weiteren bevorzugten Ausführungsform transportiert die Transporteinrichtung die Behältnisverschlüsse vereinzelt. Damit sind die transportierten Behältnisverschlüsse bevorzugt während des Transports voneinander beabstandet. Auf diese Weise ist eine vereinzelte Sterilisation der Behältnisverschlüsse möglich. Insbesondere können auf diese Weise auch Außenoberflächenbereiche der Behältnisverschlüsse zuverlässig sterilisiert werden. Bevorzugt werden die Behältnisverschlüsse in einer Reihe und insbesondere einreihig transportiert.

Erfindungsgemäß weist die Sterilisationseinrichtung eine Vielzahl von rohrförmigen Strahlungseinrichtungen auf, welche auf einem drehbaren Sterilisationsträger angeordnet ist, wobei die Strahlungseinrichtungen entlang des Transportpfads gegenüber den Behältnisverschlüssen bewegbar sind.

Bei dieser Ausgestaltung, die unten genauer beschrieben wird, wird insbesondere vorgeschlagen, dass die Sterilisationseinrichtungen bzw. Strahlfinger auf einem drehbaren eigenen Träger angeordnet sind und die Behältnisverschlüsse beispielsweise mit einem weiteren Träger beispielsweise mit einem weiteren drehbaren Träger transportiert werden. Insbesondere unterscheidet sich daher dieser Träger, auf dem die Sterilisationseinrichtungen angeordnet sind, von der Transporteinrichtung zum Transportieren der Behältnisverschlüsse. Dabei wäre es jedoch auch möglich, dass die Transporteinrichtung die Behältnisverschlüsse geradlinig transportiert.

Erfindungsgemäß weist die Transporteinrichtung einen drehbaren Träger auf sowie eine Vielzahl von an diesem Träger angeordneten Halteelementen, um die Behältnisverschlüsse gegenüber der Sterilisationseinrichtung zu bewegen. Dieser drehbare Träger unterscheidet sich dabei bevorzugt von dem oben erwähnten Sterilisationsträger.

Erfindungsgemäß fallen Drehachsen der Transporteinrichtung und der Sterilisationseinrichtung bzw. des Sterilisationsträgers auseinander. Erfindungsgemäß stehen diese Drehachse senkrecht zueinander.

Bei einer weiteren bevorzugten Ausführungsform stimmen eine Teilung der Strahlfinger und insbesondere eine Teilung der Strahlfinger in den Endabschnitten derselben und eine Teilung der Halteelemente zum Halten der Behältnisverschlüsse überein. Dabei ist es bevorzugt möglich, dass die Strahlfinger, die auf dem Sterilisationsträger angeordnet sind und die einzelnen Behältnisverschlüsse zahnradartig ineinander greifen.

Bei dieser Ausgestaltung ist es möglich, dass die Behältnisverschlüsse auf einen Stern verbracht werden und mit diesem Transportstern transportiert werden. An einer geeigneten Stelle dieses Transportsterns befindet sich eine zu diesem Transportstern (genauer zu der Drehbewegung dieses Sterns) um einen vorgegebenen Winkel, beispielsweise um 90° geneigte Scheibe, der oben erwähnte Sterilisationsträger, auf dem die Strahlungsfinger angebracht sind. Diese Scheibe dreht sich bevorzugt in der Drehrichtung des Transportsterns für die Behältnisverschlüsse mit und ist besonders bevorzugt hinsichtlich der Drehgeschwindigkeit auf diesen angepasst.

Durch die Kreisbahn der Strahlungsfinger, die in dem oben erwähnten Fall 90° zur Kreisbahn der Verschlüsse steht, können die Strahlungsfinger für einen kurzen Moment in die jeweiligen Verschlüsse eintauchen. Die Anmelderin hat ermittelt, dass zur Desinfektion eine Bestrahlungszeit von nur 0,2 Sekunden nötig ist.

Bevorzugt sind Koppelungs- und/ oder Synchronisationsmittel vorgesehen, welche die Drehbewegung der Transporteinrichtung und die Drehbewegung des Sterilisationsträgers miteinander synchronisieren. Dabei kann die Drehbewegung dieser Transporteinrichtungen mittels eines Getriebes von der Welle oder der Antriebseinheit des Transportsterns für die Kunststoffvorformlinge abgenommen werden. Es sind jedoch auch separate synchronisierte Antriebe denkbar.

Weiterhin ist es auch möglich, den Winkelbereich, in dem die Strahlung abgegeben wird, zu definieren.

Die Vorteile dieser Vorgehensweise liegen darin, dass die Behältnisverschlüsse ohne Schatten desinfiziert werden können. Die Strahlfinger können dabei in die Verschlüsse eintauchen, ohne dass dabei die Bewegung des Transportsterns für die Behältnisverschlüsse angehalten werden muss. Es ist jedoch hier nicht zwingend ein eigener Antrieb für den Sterilisationsträger nötig.

Bei einer besonders bevorzugten Ausführungsform weist die Vorrichtung eine Zuführeinrichtung auf, welche die Behältnisverschlüsse der oben erwähnten Transporteinrichtung zuführt.

Falls der oben erwähnte Transport der Behältnisverschlüsse getaktet erfolgt, wäre es möglich, dass zunächst eine Transporteinrichtung mit Verschlüssen bestückt wird. Sobald eine derartige Transporteinrichtung, beispielsweise eine Transportscheibe halb befüllt ist, kann diese Transporteinrichtung anhalten und eine Einheit, welche eine Vielzahl von Strahlungsemittern aufweist, kann die oben erwähnte Hubbewegung ausführen.

Der Hub führt dazu, dass die Strahlungseinrichtungen in den jeweiligen Behältnisverschluss eintauchen und hierbei den Verschluss vollständig sterilisieren. Wie oben erwähnt, benötigt man zur vollständigen Sterilisation etwa 0,2 Sekunden. Addiert man die Zeit für die mechanische Bewegung, ergibt sich somit eine Zeit von ca. 0,5 Sekunden. Bei einer Anlagenleistung von 36.000 Flaschen pro Stunde könnten beispielsweise fünf Strahlungseinrichtungen vorgesehen sein.

Diese Strahlungseinrichtungen könnten in ähnlicher Weise aufgebaut werden, wie aus dem Stand der Technik bekannte Strahlungseinrichtungen zur Entkeimung von Kunststoffvorformlingen.

Nach dem Wiederhochfahren der Strahlungseinrichtungen kann sich die Transporteinrichtung wieder in Bewegung setzen und durch die Rotationsbewegung die Verschlüsse in Richtung einer weiteren Einheit, wie etwa eines Verschliessers, abführen. Bei derselben Bewegung kann auch die gegenüberliegende Seite des Rotationsterns wieder mit Verschlüssen befüllt werden und der Vorgang wiederholt sich.

Zur Außenbehandlung kann wie oben erwähnt ein stationärer Finger etwa in einem Rinnenzulauf installiert werden. Die erwähnte Transportscheibe und der Fingeraustritt befinden sich dabei besonders bevorzugt in einer strahlendichten Kammer.

Bei einer weiteren bevorzugten Ausführungsform weist die Vorrichtung einen Reinraum auf, der insbesondere denjenigen Bereich, in dem die Sterilisation stattfindet und oder den Transportpfad der Behältnisverschlüsse von einer Umgebung und insbesondere einer unsterilen Umgebung abtrennt. Dabei kann dieser Reinraum bezüglich einander bewegliche Wandungen aufweisen, die jedoch abgedichtet sind, beispielsweise mit Labyrinthdichtungen oder sogenannten Wasserschlössern. Damit findet bevorzugt die Sterilisation der Behältnisverschlüsse innerhalb eines Reinraums statt.

Bei einer weiteren bevorzugten Ausführungsform weist die Vorrichtung eine Abschirmeinheit auf, welche im Wege der Sterilisation entstehende Strahlung und insbesondere Röntgenstrahlung abschirmt.

Dabei kann eine derartige Abschirmeinrichtung beispielsweise um die einzelnen Sterilisationseinrichtungen bzw. Strahlfinger aufgebaut sein.

Die vorliegende Erfindung ist weiterhin auf ein Verfahren zum Sterilisieren von Behältnisverschlüssen gerichtet, wobei die Behältnisverschlüsse mit einer Transporteinrichtung entlang eines vorgegebenen Transportpfads transportiert werden und mit einer Sterilisationseinrichtung die Behältnisverschlüsse wenigstens abschnittsweise sterilisiert werden und bevorzugt wenigstens abschnittsweise in einem Innenraum der Behältnisverschlüsse sterilisiert werden.

Erfindungsgemäß weist die Sterilisationseinrichtung eine Ladungsträgerquelle auf, welche Ladungsträger erzeugt und eine Strahlungseinrichtung strahlt die Ladungsträger auf eine zu sterilisierende Oberfläche der Behältnisverschlüsse ein, wobei die Strahlungseinrichtung genauer wenigstens ein Element dieser Strahlungseinrichtung wenigstens zeitweise in einen Innenraum der Behältnisverschlüsse eingeführt wird bzw. eintaucht.

Insbesondere taucht wenigstens ein Abschnitt der oben erwähnten Strahlfinger und insbesondere ein Endabschnitt dieser Strahlfinger in die Behältnisverschlüsse ein.

Es wird daher auch verfahrensseitig vorgeschlagen, dass die Strahlungseinrichtung und erfindungsgemäß ein Endabschnitt der Strahlungseinrichtung zum Sterilisieren in einen Innenraum der Behältnisverschlüsse eintaucht. Auf diese Weise ist eine effiziente Innensterilisation der Behältnisverschlüsse möglich.

Bevorzugt werden die Sterilisationseinrichtungen auf einem kreisförmigen Pfad transportiert. Bevorzugt weicht dabei der Transportpfad dieser Sterilisationseinrichtungen von dem Transportpfad der Behältnisverschlüsse während deren Sterilisation ab.

Weitere Vorteile und Ausführungsformen ergeben sich aus den beigefügten Zeichnungen:
Darin zeigen:
- Fig. 1: eine schematische Darstellung einer nicht erfindungsgemäßen Vorrichtung in einer ersten Ausführungsform;
- Fig. 2: die Vorrichtung aus Figur 1 in einer weiteren Darstellung;
- Fig. 3: eine erfindungsgemäße Vorrichtung in einer weiteren Ausführungsform; und
- Fig. 4: eine Darstellung eines Behältnisverschlusses mit einer Strahleinrichtung.

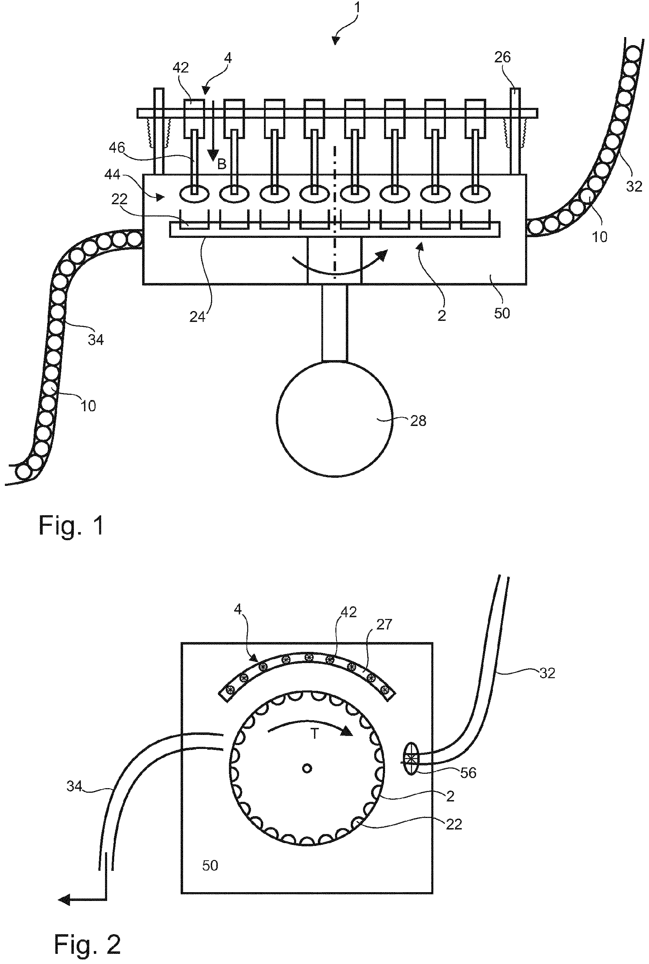

Figur 1 zeigt eine nicht erfindungsgemäße Vorrichtung 1 zum Sterilisieren von Behältnisverschlüssen 10. Dabei werden diese Behältnisverschlüsse 10 über eine Zuführeinrichtung, wie etwa eine Zuführrinne 32 einer Transporteinrichtung 2 zugeführt. Diese Transporteinrichtung 2 weist einen drehbaren Träger 24 auf, der eine Vielzahl von Halteelementen zum Halten der Behältnisverschlüsse 10 aufweist. Die Behältnisverschlüsse 10 werden zunächst mit der Transporteinrichtung 2 eingefahren, bis beispielsweise die einzelnen Halteelemente besetzt sind.

Oberhalb der Behältnisverschlüsse 10 bzw. der Transporteinrichtung 2 ist eine in ihrer Gesamtheit mit 4 bezeichnete Sterilisationseinrichtung dargestellt. Diese weist eine Vielzahl von Ladungsträgerquellen 42 auf. Das Bezugszeichen 46 bezieht sich auf einen rohrförmigen Körper innerhalb dessen die Ladungsträger, d.h. hier die Elektronen in Richtung eines Austrittsfensters beschleunigt werden. Das Bezugszeichen 44 bezieht sich entsprechend auf die einzelnen Strahlungseinrichtungen, aus denen die Elektronen austreten, die zur Beaufschlagung der Behältnisverschlüsse dienen.

Weiterhin ist ein Träger 26 vorgesehen, an dem die einzelnen Sterilisationseinrichtungen bzw. Strahlfinger 44 angeordnet sind. Dieser Träger 26 erlaubt ein Bewegen der einzelnen Strahlfinger 46 und damit auch der Strahlungseinrichtungen 44 in der Bewegungsrichtung B.

Der Transport der Behältnisverschlüsse erfolgt hier getaktet mittels einer Antriebseinrichtung wie einem Motor 28. Sobald die Behältnisverschlüsse in ihrer Sterilisationsposition sind, werden die Strahlfinger 46 mit der Hubeinrichtung bzw. dem Träger 26 abgesenkt und in den Innenraum 10a der Behältnisverschlüsse eingetaucht. Dort erfolgt eine Sterilisation.

Anschließend werden die Strahlfinger 46 wieder angehoben und die Behältnisverschlüsse von der Transporteinrichtung 2 abgeführt, was hier insbesondere mittels einer Abführrinne 34 erfolgen kann.

Das Bezugszeichen 50 kennzeichnet einen Reinraum, der den Sterilisationsbereich umgibt. Dieser Reinraum 50 kann dabei Schleusen aufweisen, über welche die Behältnisverschlüsse zugeführt werden und über welche sie auch wieder ausgeführt werden. Weiterhin ist es denkbar, dass der Reinraum gegenüber dem atmosphärischen Druck mit einem Überdruck beaufschlagt wird, sodass das Eintreten von Keimen in den Reinraum sicher verhindert werden kann.

Weiterhin weist die Vorrichtung eine (nicht gezeigte) Abschirmeinrichtung auf, welche insbesondere die elektromagnetische Strahlung und insbesondere Röntgenstrahlung abschirmt, welche durch die einzelnen Ladungsträgerquellen 42 bzw. die Strahlungseinrichtungen 44 erzeugt wird.

Figur 2 zeigt eine Draufsicht auf die in Figur 1 gezeigte Vorrichtung. Man erkennt hier, dass die Transporteinrichtung 2 als drehbarer Träger ausgeführt ist und eine Vielzahl von Halteelementen 22 zum Aufnehmen der Behältnisverschlüsse 10 aufweist. Auch die einzelnen Strahlungseinrichtungen bzw. Strahlfinger 44 sind auf einem gemeinsamen Träger 27 angeordnet, der in seiner Gesamtheit in Richtung des Transportpfads der Behältnisverschlüsse absenkbar und wieder hebbar ist.

Das Bezugszeichen 56 kennzeichnet eine weitere Sterilisationseinrichtung, die insbesondere zur Außensterilisation der Behältnisverschlüsse dient. Diese kann beispielsweise unterhalb der besagten Zuführrinne 32 angeordnet sein.

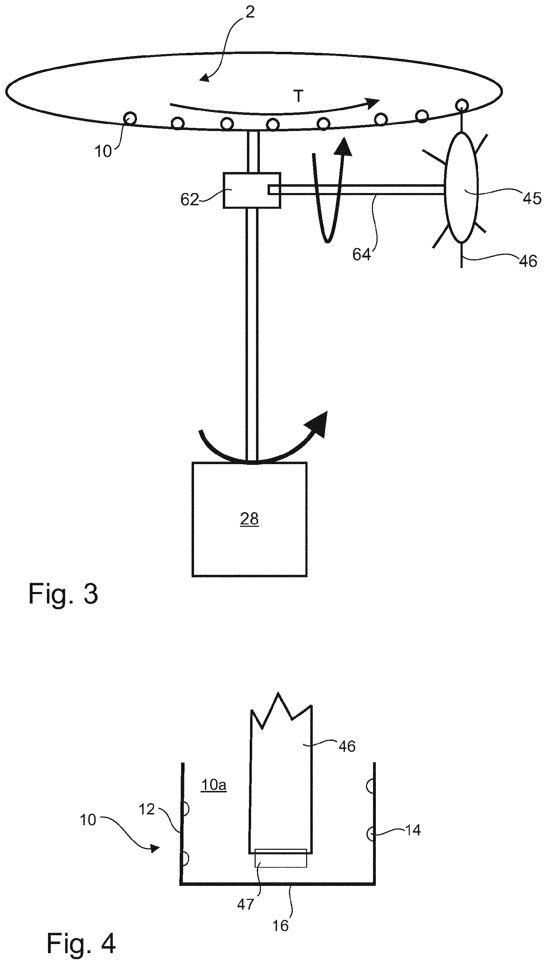

Figur 3 zeigt schematisch eine weitere Ausgestaltung der vorliegenden Erfindung. Auch hier ist wieder eine Transporteinrichtung 2 vorgesehen, welche eine Vielzahl von Behältnisverschlüssen 10 entlang des Transportpfads T bewegt. Zusätzlich ist ein Sterilisationsträger 45 vorgesehen, an dem eine Vielzahl von Strahlfingern 46 angeordnet ist.

Das Bezugszeichen 62 kennzeichnet ein Getriebe, mit welchem eine Antriebswelle 64, an der der Sterilisationsträger 45 angeordnet ist, mit der Transporteinrichtung 2 synchronisiert wird, sodass die einzelnen Strahlfinger jeweils in die einzelnen Behältnisverschlüsse eindringen können.

Es wäre auch möglich, dass der Sterilisationsträger anders als hier gezeigt oberhalb der Behältnisverschlüsse angeordnet ist.

Das Bezugszeichen 28 kennzeichnet wieder die Antriebseinrichtung zum Antreiben der Transporteinrichtung 2 und damit hier auch der Welle 64.

Fig. 4 zeigt eine Darstellung eines Behältnisverschlusses 10 auf. Dieser weist eine Deckelfläche 16 auf, sowie eine Umfangswandung 12, an der ein Innengewinde angeordnet ist. Das Bezugszeichen 10a kennzeichnet den Innenbereich des Behältnisverschlusses.

Das Bezugszeichen 46 kennzeichnet einen Strahlfinger, der gerade in den Behältnisverschluss 10 eingetaucht ist. Das Bezugszeichen 47 kennzeichnet ein am unteren Ende des Strahlfingers angeordnetes Austrittsfenster, über welches die Elektronen aus dem Strahlfinger 46 austreten können Es wird darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann.

### Bezugszeichenliste

- 1: Vorrichtung
- 2: Transporteinrichtung
- 4: Sterilisationseinrichtung
- 10: Behältnisverschlüsse
- 10a: Innenraum der Behältnisverschlüsse
- 12: Umfangswandung
- 14: Gewinde
- 16: Deckel
- 22: Halteelemente
- 24: drehbarer Träger
- 26: Träger
- 27: Träger
- 28: Antriebeinrichtung, Motor
- 32: Zuführrinne
- 34: Abführrinne
- 42: Ladungsträgerquellen
- 44: Strahlungseinrichtung
- 45: Sterilisationsträger
- 46: Strahlfinger, rohrförmiger Körper
- 47: Austrittsfenster
- 50: Reinraum
- 62: Getriebe
- 64: Antriebswelle
- T: Transportpfad
- B: Bewegung der Strahlfinger

## Patentansprüche

1. Vorrichtung (1) zum Sterilisieren von Behältnisverschlüssen (10) mit einer Transporteinrichtung (2), welche die Behältnisverschlüsse (10) entlang eines vorgegebenen Transportpfads (T) transportiert und mit einer Sterilisationseinrichtung (4) welche dazu geeignet und bestimmt ist, die Behältnisverschlüsse (10) wenigstens abschnittsweise zu sterilisieren, wobei die Sterilisationseinrichtung (4) eine Ladungsträgerquelle (42) aufweist, welche Ladungsträger erzeugt und eine Strahlungseinrichtung (44), welche die Ladungsträger auf eine zu sterilisierende Oberfläche der Behältnisverschlüsse einstrahlt, wobei wenigstens ein Endabschnitt der Sterilisationseinrichtung in einen Innenbereich (10a) der Behältnisverschlüsse (10) einführbar ist, und die Transporteinrichtung (2) einen drehbaren Träger (24) aufweist sowie eine Vielzahl von an diesem Träger (24) angeordneten Halteelementen (22), um die Behältnisverschlüsse gegenüber der Sterilisationseinrichtung (4) zu bewegen,
**dadurch gekennzeichnet, dass**
die Sterilisationseinrichtung (4) eine Vielzahl von rohrförmigen Strahlungseinrichtungen aufweist, welche auf einem drehbaren Sterilisationsträger (45) angeordnet ist, wobei die Strahlungseinrichtungen entlang des Transportpfads gegenüber den Behältnisverschlüssen (10) bewegbar sind, wobei Drehachsen der Transporteinrichtung und des Sterilisationsträgers auseinanderfallen und senkrecht zueinander stehen.

2. Vorrichtung (1) nach Anspruch 1,
**dadurch gekennzeichnet, dass**
die Sterilisationseinrichtung (4) einen rohrförmigen Körper (46) aufweist, innerhalb dessen die Ladungsträger beschleunigt werden.

3. Vorrichtung (1) nach Anspruch 1,
**dadurch gekennzeichnet, dass**
die Sterilisationseinrichtung (4) eine Strahlungseinrichtung (44) aufweist, welche gegenüber den Behältnisverschlüssen in einer Bewegungsrichtung (B) bewegbar ist, welche zumindest eine Komponente aufweist, welche senkrecht zu dem Transportpfad der Behältnisverschlüsse steht.

4. Vorrichtung (1) nach wenigstens einem der vorangegangenen Ansprüche,
**dadurch gekennzeichnet, dass**
die Sterilisationseinrichtung (4) eine Vielzahl von entlang des Transportpfads (T) hintereinander angeordneten rohrförmigen Körpern (46) aufweist.

5. Vorrichtung (1) nach wenigstens einem der vorangegangenen Ansprüche,
**dadurch gekennzeichnet, dass**
die Sterilisationseinrichtung (4) stationär entlang des Transportpfads (T) der Behältnisverschlüsse (10) angeordnet ist.

6. Vorrichtung (1) nach wenigstens einem der vorangegangenen Ansprüche,
**dadurch gekennzeichnet, dass**
die Sterilisationseinrichtung (4) und/oder der rohrförmige Körper beweglich in der Transportrichtung der Behältnisverschlüsse angeordnet ist.

7. Vorrichtung (1) nach wenigstens einem der vorangegangenen Ansprüche,
**dadurch gekennzeichnet, dass**
die Transporteinrichtung (2) die Behältnisverschlüsse vereinzelt transportiert.

8. Verfahren zum Sterilisieren von Behältnisverschlüssen (10), wobei die Behältnisverschlüsse mit einer Transporteinrichtung (2) entlang eines vorgegebenen Transportpfads (T) transportiert werden und mit einer Sterilisationseinrichtung (4) die Behältnisverschlüsse (10) wenigstens abschnittsweise sterilisiert werden, wobei die Sterilisationseinrichtung (4) eine Ladungsträgerquelle (42) aufweist, welche Ladungsträger erzeugt und eine Strahlungseinrichtung (44) die Ladungsträger auf eine zu sterilisierende Oberfläche der Behältnisverschlüsse (10) einstrahlt, wobei ein Endabschnitt der Strahlungseinrichtung (44) wenigstens zeitweise in einen Innenraum der Behältnisverschlüsse (10) eintaucht, und die Transporteinrichtung (2) einen drehbaren Träger (24) aufweist sowie eine Vielzahl von an diesem Träger (24) angeordneten Halteelementen (22), um die Behältnisverschlüsse gegenüber der Sterilisationseinrichtung (4) zu bewegen,
**dadurch gekennzeichnet, dass**
die Sterilisationseinrichtung (4) eine Vielzahl von rohrförmigen Strahlungseinrichtungen aufweist, welche auf einem drehbaren Sterilisationsträger (45) angeordnet ist, wobei die Strahlungseinrichtungen entlang des Transportpfads gegenüber den Behältnisverschlüssen (10) bewegbar sind, wobei Drehachsen der Transporteinrichtung und des Sterilisationsträgers auseinanderfallen und senkrecht zueinander stehen.

## Claims

1. Apparatus (1) for sterilising container closures (10) with a transport device (2) which transports the container closures (10) along a predetermined transport path (T), and with a sterilisation device (4) which is suitable and intended for sterilising the container closures (10) at least in sections, wherein the sterilisation device (4) has a source of charge carriers (42) which generates charge carriers and a radiation device (44) which irradiates the charge carriers onto a surface of the container closures to be sterilised, wherein at least one end portion of the sterilisation device can be introduced into an inner region (10a) of the container closures (10), and the transport device (2) has a rotatable support (24) as well as a plurality of holding elements (22) arranged on this support (24) in order to move the container closures relative to the sterilisation device (4),
**characterised in that**
the sterilisation device (4) has a plurality of tubular radiation devices which are arranged on a rotatable sterilisation support (45), wherein the radiation devices are movable along the transport path relative to the container closures (10), wherein axes of rotation of the transport device and of the sterilisation support do not coincide and are perpendicular to one another.

2. Apparatus (1) according to claim 1,
**characterised in that**
the sterilisation device (4) has a tubular body (46), inside which the charge carriers are accelerated.

3. Apparatus (1) according to claim 1,
**characterised in that**
the sterilisation device (4) has a radiation device (44) which is movable relative to the container closures in a direction of movement (B) which has at least one component which is perpendicular to the transport path of the container closures.

4. Apparatus (1) according to at least one of the preceding claims,
**characterised in that**
the sterilisation device (4) has a plurality of tubular bodies (46) arranged one behind the other along the transport path (T).

5. Apparatus (1) according to at least one of the preceding claims,
**characterised in that**
the sterilisation device (4) is arranged in a stationary manner along the transport path (T) of the container closures (10).

6. Apparatus (1) according to at least one of the preceding claims,
**characterised in that**
the sterilisation device (4) and/or the tubular body is arranged movably in the transport direction of the container closures.

7. Apparatus (1) according to at least one of the preceding claims,
**characterised in that**
the transport device (2) transports the container closures individually.

8. Method for sterilising container closures (10), wherein the container closures are transported by a transport device (2) along a predetermined transport path (T) and the container closures (10) are sterilised at least in sections by a sterilisation device (4), wherein the sterilisation device (4) has a source of charge carriers (42) which generates charge carriers and a radiation device (44) which radiates the charge carriers onto a surface of the container closures (10) to be sterilised, wherein an end portion of the sterilisation device (44) protrudes at least temporarily into an interior of the container closures (10), and the transport device (2) has a rotatable support (24) as well as a plurality of holding elements (22) arranged on this support (24) in order to move the container closures relative to the sterilisation device (4),
**characterised in that**
the sterilisation device (4) has a plurality of tubular radiation devices which are arranged on a rotatable sterilisation support (45), wherein the radiation devices are movable along the transport path relative to the container closures (10), wherein axes of rotation of the transport device and of the sterilisation support do not coincide and are perpendicular to one another.

## Revendications

1. Dispositif (1) pour stériliser des fermetures de récipients (10) avec un système de transport (2), lequel transporte les fermetures de récipients (10) le long d'un trajet de transport (T) prédéfini et avec un système de stérilisation (4), lequel est adapté et destiné à stériliser les fermetures de récipients (10) au moins sur certaines parties, dans lequel le système de stérilisation (4) présente une source de porteurs de charge (42), laquelle produit des porteurs de charge et un système d'irradiation (44), lequel irradie les porteurs de charge sur une surface à stériliser des fermetures de récipients, dans lequel au moins une partie d'extrémité du système de stérilisation peut être introduite dans une zone intérieure (10a) des fermetures de récipients (10), et le système de transport (2) présente un support (24) rotatif ainsi qu'une pluralité d'éléments de retenue (22) disposés sur ce support (24), afin de déplacer les fermetures de récipients par rapport au système de stérilisation (4),
**caractérisé en ce que**
le système de stérilisation (4) présente une pluralité de systèmes d'irradiation tubulaires, laquelle est disposée sur un support de stérilisation (45) rotatif, dans lequel les systèmes d'irradiation sont mobiles le long du trajet de transport par rapport aux fermetures de récipients (10), dans lequel les axes de rotation du système de transport et du support de stérilisation se séparent et sont perpendiculaires l'un à l'autre.

2. Dispositif (1) selon la revendication 1,
**caractérisé en ce que**
le système de stérilisation (4) présente un corps tubulaire (46), à l'intérieur duquel les porteurs de charge sont accélérés.

3. Dispositif (1) selon la revendication 1,
**caractérisé en ce que**
le système de stérilisation (4) présente un système d'irradiation (44), lequel est mobile par rapport aux fermetures de récipients dans une direction de mouvement (B), laquelle présente au moins une composante, laquelle est perpendiculaire au trajet de transport des fermetures de récipients.

4. Dispositif (1) selon au moins l'une des revendications précédentes, **caractérisé en ce que**
le système de stérilisation (4) présente une pluralité de corps tubulaires (46) disposés les uns derrière les autres le long du trajet de transport (T).

5. Dispositif (1) selon au moins l'une des revendications précédentes, **caractérisé en ce que**
le système de stérilisation (4) est disposé de manière fixe le long du trajet de transport (T) des fermetures de récipients (10).

6. Dispositif (1) selon au moins l'une des revendications précédentes, **caractérisé en ce que**
le système de stérilisation (4) et/ou le corps tubulaire est disposé de manière mobile dans la direction de transport des fermetures de récipients.

7. Dispositif (1) selon au moins l'une des revendications précédentes, **caractérisé en ce que**
le système de transport (2) transporte les fermetures de récipients une à une.

8. Procédé pour stériliser des fermetures de récipients (10), dans lequel les fermetures de récipients sont transportées avec un système de transport (2) le long d'un trajet de transport (T) prédéfini et les fermetures de récipients (10) sont stérilisées au moins sur certaines parties avec un système de stérilisation (4), dans lequel le système de stérilisation (4) présente une source de porteurs de charge (42), laquelle produit des porteurs de charge et un système d'irradiation (44) irradie les porteurs de charge sur une surface à stériliser des fermetures de récipients (10), dans lequel une partie d'extrémité du système d'irradiation (44) s'enfonce au moins par intermittence dans un espace intérieur des fermetures de récipients (10), et le système de transport (2) présente un support (24) rotatif ainsi qu'une pluralité d'éléments de retenue (22) disposés sur ce support (24), afin de déplacer les fermetures de récipients par rapport au système de stérilisation (4),
**caractérisé en ce que**
le système de stérilisation (4) présente une pluralité de systèmes d'irradiation tubulaires, laquelle est disposée sur un support de stérilisation (45) rotatif, dans lequel les systèmes d'irradiation sont mobiles le long du trajet de transport par rapport aux fermetures de récipients (10), dans lequel les axes de rotation du système de transport et du support de stérilisation se séparent et sont perpendiculaires l'un à l'autre.
